## Europäisches Patentamt
## European Patent Office
## Office européen des brevets

(19)

(11) Veröffentlichungsnummer: **0 013 859**
**A1**

(12)

# EUROPÄISCHE PATENTANMELDUNG

(21) Anmeldenummer: 79710016.1

(22) Anmeldetag: 14.12.79

(51) Int. Cl.³: **C 07 D 235/26**

(30) Priorität: 21.12.78 DE 2855226

(43) Veröffentlichungstag der Anmeldung: 06.08.80
Patentblatt 80/16

(84) Benannte Vertragsstaaten: **CH DE FR GB IT**

(71) Anmelder: **HOECHST AKTIENGESELLSCHAFT, Zentrale Patentabteilung Postfach 80 03 20, D-6230 Frankfurt/Main 80 (DE)**

(72) Erfinder: **Heise, Hartmut, Dr., Am Rehsteig 8, D-6232 Bad Soden am Taunus (DE)**
Erfinder: **Hille, Ernst, Dr., Im Herlenstück 15, D-6233 Kelkheim (Taunus) (DE)**
Erfinder: **Wagner, Reinhard, Dr., Buchenweg 28, D-6200 Wiesbaden (DE)**
Erfinder: **Kümmerle, Kurt, Dr., Unter den Eichen 1, D-6233 Kelkheim (Taunus) (DE)**

(54) Verfahren zur Herstellung von Benzimidazolon-(2).

(57) Die Kondensation von o-Phenylendiamin mit Harnstoff in Wasser liefert Benzimidazolon-(2) in guter Ausbeute und hoher Reinheit, wenn die Reaktion bei pH 4–9 und mindestens 90 °C erfolgt. Es kann mit einem minimalen Harnstoffüberschuß und drucklos gearbeitet werden.

EP 0 013 859 A1

HOECHST AKTIENGESELLSCHAFT    HOE 78/F 277        Dr.KL/ss

Verfahren zur Herstellung von Benzimidazolon-(2)

Aus der DE-PS 20 52 026 ist es bekannt, o-Phenylendiamin mit überschüssigem Harnstoff in Wasser bei 90 bis 160°C zu Benzimidazolon-(2) umzusetzen. Diese Umsetzung mit Harnstoff im Reaktionsmedium Wasser bringt gegenüber bekannten Verfahren in anderen Lösemitteln bzw. in der Schmelze und auch gegenüber Verfahren, die vom Phosgen oder Isocyanaten ausgehen, eine Reihe von Vorteilen mit sich. Allerdings erfordert die Umsetzung bei Temperaturen über dem Siedepunkt Druckgefäße und besondere Vorkehrungen gegen Korrosion durch das heiße alkalische Reaktionsmedium, das durch die teilweise Zersetzung des Harnstoffs entsteht.

Es wurde nun gefunden, daß man Benzimidazolon-(2) durch Erhitzen von o-Phenylendiamin und Harnstoff in Wasser auf eine Temperatur von mindestens 90, vorzugsweise mindestens 100°C besonders vorteilhaft erhält, wenn die Reaktion im pH-Bereich von 4 bis 9, vorzugsweise 4 bis 8, insbesondere 4,5 bis 7 durchgeführt wird.

Überraschenderweise kann bei dem erfindungsgemäßen Verfahren auf die Anwendung von Druck verzichtet werden, da bereits bei den Temperaturen von 90° C bis zum Siedepunkt der Reaktionsmischung hohe Umsätze erzielt werden. Ein weiterer Vorteil ist, daß erfindungsgemäß nur ein minimaler Harnstoffüberschuß erforderlich ist von vorzugsweise bis zu 1,5 Mol pro Mol o-Phenylendiamin. Diese Tatsache ist außerordentlich überraschend, da aus Ullmanns Enzyklopädie der technischen Chemie, 3. Aufl., 8. Band, S. 380 bekannt war, daß das Kochen von Harnstoff mit Alkalien oder Säuren den Zerfall in Ammoniak und Kohlendioxid bewirkt.

Der erfindungsgemäße pH-Bereich von 4 bis 9 wird mit Säuren eingestellt, wobei im Prinzip jede beliebige Säure in Betracht kommt, soweit sie mit den Reaktionsteilnehmern keine unerwünschten Nebenreaktionen eingeht. Zweckmäßig werden Mineralsäuren wie Phosphorsäure, insbesondere Salzsäure oder Schwefelsäure eingesetzt.

Durch die Säurezugabe wird das frei werdende Ammoniak als Salz gebunden, was zu einer Erhöhung der Siedetemperatur auf ca. 105°C führt, so daß ein Druckgefäß entbehrlich ist. Es ist so auch leichter möglich, den Umsetzungsgrad durch Probenentnahme zu jedem beliebigen Zeitpunkt der Reaktion zu bestimmen und beispielsweise die Ausbeute in Abhängigkeit von der Harnstoffzugabe oder Säurezugabe zu steuern.

Eine vorteilhafte Ausführungsform des erfindungsgemäßen Verfahrens besteht darin, daß man das o-Phenylendiamin und einen Teil des Harnstoffs vorlegt und weiteren Harnstoff und die Säure gleichzeitig zudosiert. Vorteilhaft wird hierbei am Anfang eine geringere Menge Harnstoff als die stöchiometrisch erforderliche vorgelegt. Eine weitere günstige Reaktionsführung besteht darin, daß man das o-Phenylendiamin in Wasser vorlegt und Harnstoff und Säure zudosiert. In jedem Falle kann die Zugabe einer Reaktionskomponente kontinuierlich oder absatzweise erfolgen.

Vorteilhaft wird gegen Ende der Reaktion ein pH-Wert von mehr als 5, vorzugsweise mehr als 6 eingestellt, da hierdurch Reinheit und Kristallstruktur des Produktes günstig beeinflußt werden.

Das o-Phenylendiamin kann in fester Form oder als wäßrige Lösung zugesetzt werden, wie sie beispielsweise bei der Reduktion des o-Nitranilins erhalten wird.

Da das o-Phenylendiamin oxydationsempfindlich ist, empfiehlt es sich, die Umsetzung unter Sauerstoffausschluß durchzuführen. Die Umsetzung wird deshalb zweckmäßig unter Inertgasschutz durchgeführt, wobei als Inertgas Kohlendioxid und Stickstoff bevorzugt sind. An Stelle des Inertgasschutzes oder zusätzlich zum Inertgasschutz kann auch ein geeignetes Reduktionsmittel dem Reaktionsgemisch zugesetzt werden, beispielsweise Natriumdithionit, Natriumsulfit oder -hydrogensulfit.

Das Verfahrensprodukt fällt in hoher Reinheit an und kann ohne Umfällung oder Umkristallisation als Zwischenprodukt für Farbstoffe und Pigmente eingesetzt werden.

In den folgenden Beispielen beziehen sich Teile und Prozente auf das Gewicht, sofern nichts anderes angegeben ist.

Beispiel 1

In einem Emailkessel werden unter Stickstoff 2000 Teile Trinkwasser, 700 Teile o-Phenylendiamin und 300 Teile Harnstoff vorgelegt und auf 100° - 105°C erhitzt. Nun läßt man soviel 95 %ige Schwefelsäure zulaufen, daß der pH-Wert immer zwischen 5 und 6 gehalten wird. Nach 3 und 6 Stunden werden jeweils nochmals 100 Teile Harnstoff zugegeben. Der pH-Wert wird weiter bei 5 - 6 gehalten.

Man entnimmt eine Probe und prüft durch Titration auf noch vorhandenes o-Phenylendiamin. Wir weniger als 0,2 % gefunden, so stellt man mit Lauge neutral und läßt noch eine Stunde rühren. Dann isoliert man das Produkt auf einer Nutsche und wäscht mit warmem Wasser salzfrei. Ausbeute: 834 Teile = 96 % d. Theorie. Schmelzpunkt: 314 - 315°C.

Das Produkt zeigte im Dünnschichtchromatogramm keine Verunreinigungen.

Beispiel 2

Man verfährt wie in Beispiel 1 beschrieben, nur werden beim Vorlegen von Harnstoff 40 Teile Natriumdithionit und bei jeder weiteren Zugabe von Harnstoff 15 Teile Natriumdithionit zugegeben.

Ausbeute und Schmelzpunkt sind wie in Beispiel 1.

Die Qualität der in Beispiel 1 und 2 beschriebenen Produkte unterscheidet sich darin, daß eine mit verdünnter Natronlauge hergestellte ca. 30 %ige Lösung auf einem Filterpapier einige Krümel eines schwarz-bräunlichen, geringen Niederschlages hinterläßt (Beispiel 1), während bei Beispiel 2 dieser weitgehend verschwunden ist. Bei der Herstellung hochwertiger Produkte sind diese dunkelgefärbten Nebenprodukte unerwünscht.

Beispiel 3

Man legt die in Beispiel 1 beschriebene Menge an Trinkwasser und o-Phenylendiamin vor, heizt auf und dosiert über eine mit Stickstoff gespülte Schnecke in ca. 6 Stunden 450 Teile Harnstoff zu. Um Oxydationsprozesse zu vermeiden, ist es zweckmäßig, dem Harnstoff bis zu 30 Teile Natriumdithionit zuzumischen.

Gleichzeitig läuft Schwefelsäure in dem Maße zu, daß der pH-Wert zwischen 5 und 6 gehalten wird.

Nach beendeter Zugabe prüft man in der beschriebenen Weise, ob die Reaktion beendet ist und verfährt, falls dies positiv ist, in der in Beispiel 1 beschriebenen Weise. Die Ausbeuten liegen zwischen 94 und 97 %. Der Schmelzpunkt ist wie in Beispiel 1.

Patentansprüche:

1. Verfahren zur Herstellung von Benzimidazolon-(2) durch Erhitzen von o-Phenylendiamin und Harnstoff in Wasser auf eine Temperatur von mindestens 90°C, dadurch gekennzeichnet, daß die Reaktion bei einem pH-Wert von 4 bis 9 durchgeführt wird.

2. Verfahren nach Anspruch 1, dadurch gekennzeichnet, daß die Reaktion in einem pH-Bereich von 4 bis 8 durchgeführt wird.

3. Verfahren nach Anspruch 1 und 2, dadurch gekennzeichnet, daß die Reaktion in einem pH-Bereich von 4,5 bis 7 durchgeführt wird.

4. Verfahren nach Anspruch 1 bis 3, dadurch gekennzeichnet, daß die Reaktion drucklos durchgeführt wird.

5. Verfahren nach Anspruch 1 bis 4, dadurch gekennzeichnet, daß das Molverhältnis von o-Phenylendiamin zu Harnstoff nicht größer als 1 zu 1,5 ist.

6. Verfahren nach Anspruch 1 bis 5, dadurch gekennzeichnet, daß man o-Phenylendiamin und einen Teil des Harnstoffs vorlegt und weiteren Harnstoff und Säure zudosiert.

7. Verfahren nach Anspruch 1 bis 5, dadurch gekennzeichnet, daß man das o-Phenylendiamin in Wasser vorlegt und Harnstoff und Säure zudosiert.

8. Verfahren nach Anspruch 1 bis 7, dadurch gekennzeichnet, daß man am Ende der Reaktion einen pH-Wert von mehr als 5 einstellt.

9. Verfahren nach Anspruch 1 bis 8, dadurch gekennzeichnet, daß man die Reaktion unter Sauerstoffausschluß durchführt.

10. Verfahren nach Anspruch 1 bis 9, dadurch gekennzeichnet, daß man dem Reaktionsgemisch ein Reduktionsmittel zusetzt.

Europäisches
Patentamt

**EUROPÄISCHER RECHERCHENBERICHT**

0013859
Nummer der Anmeldung

EP 79 71 0016

| Kategorie | Kennzeichnung des Dokuments mit Angabe, soweit erforderlich, der maßgeblichen Teile | betrifft Anspruch |
|---|---|---|
| | DE - A - 2 131 367 (HOECHST) | 1-3,5, 8 |
| | ✱ Seiten 1-4,8; Beispiel 6 ✱ | |
| | -- | |
| | DE - B - 1 115 740 (PARKE DAVIS & CO) | 1-3,5 |
| | ✱ Seite 1 ✱ | |
| | ---- | |

**EINSCHLÄGIGE DOKUMENTE**

**KLASSIFIKATION DER ANMELDUNG (Int.Cl. 3)**

C 07 D 235/26

**RECHERCHIERTE SACHGEBIETE (Int. Cl. 3)**

C 07 D 235/26

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 16-04-1980 | DE BUYSER |

EPA form 1503.1   06.78